# FASCICULE DE BREVET EUROPEEN

(11) **EP 0 418 109 B1**
(45) Date de publication et mention de la délivrance du brevet: **18.01.1995**
(21) Numéro de dépôt: 90402364.5
(22) Date de dépôt: 24.08.1990
(51) Int. Cl.: A61B 1/00, G02B 21/18

(54) **Ensemble microscope-endoscope utile notamment en chirurgie**
Mikroskop/Endoskop-Vorrichtung, insbesondere zur Verwendung in der Chirurgie
Microscope/endoscope assembly, particularly useful in surgery

(30) Priorité: 12.09.1989 FR 8911914
(43) Date de publication de la demande: 20.03.1991
(73) Titulaire: Leon, Claude, F-20144 Sainte Lucie de Porto Vecchio (FR); Leon, Joseph, F-20144 Sainte Lucie de Porto Vecchio (FR); Leon, Jean-Marie, F-2017 Porto Vecchio (FR)
(72) Inventeur: Leon, Claude, F-20144 Sainte Lucie de Porto Vecchio (FR); Leon, Joseph, F-20144 Sainte Lucie de Porto Vecchio (FR); Leon, Jean-Marie, F-2017 Porto Vecchio (FR)
(74) Mandataire: Phélip, Bruno

(56) Documents cités:
- WO-A-88/04786
- DE-A- 3 610 024
- FR-A- 2 513 772
- GB-A- 2 140 578

## Description

La présente invention concerne un ensemble microscope-endoscope utile notamment en chirurgie.

Dans tous les domaines, la chirurgie a fait d'énormes progrès au cours de ces dernières années.

Les chirurgiens doivent utiliser des appareils de plus en plus sophistiqués tant pour les diagnostics que la thérapeutique.

Dans certains domaines, les praticiens utilisent deux appareils, d'une part le microscope et d'autre part l'endoscope. Un exemple typique réside, par exemple, en chirurgie oculaire.

Chaque appareil a une fonction propre.

Le microscope opératoire ne permet qu'une vision frontale des éléments intra-oculaires rendant totalement impossible la vision des régions angulaires, c'est-à-dire les vues de profil.

L'endoscope permet la vision des régions angulaires et l'introduction de moyens thérapeutiques.

L'utilisation d'un appareil, par exemple, le microscope, puis de l'autre appareil, l'endoscope ne permet pas la détermination précise désirée.

Lors de l'utilisation séquentielle du microscope et de l'endoscope, le chirurgien doit poser ses yeux alternativement sur les deux oculaires de chaque appareil, cela n'est pas aisé et ne permet pas la mise en oeuvre de certaines opérations particulières.

Il existe donc un besoin d'un appareil permettant au chirurgien d'observer et de travailler sans que ses yeux quittent les oculaires d'un seul appareil.

La présente invention répond au besoin précité en proposant un ensemble compact qui permet au chirurgien de réaliser toutes les opérations nécessaires dans son art sans que ses yeux ne quittent les oculaires du microscope.

L'appareil selon la présente invention permet trois types d'observations.

Dans une première position, le chirurgien ou l'utilisateur utilise la voie optique du microscope.

Dans une deuxième position, le chirurgien utilise la voie optique de l'endoscope mais il voit par l'intermédiaire des oculaires du microscope. Il n'a donc pas à se mouvoir et ses mains sont libres, ceci est particulièrement important dans certains domaines particuliers comme la chirurgie oculaire quand on sait que l'endoscope est utilisé comme un crayon en l'orientant dans toutes les directions nécessaires à la bonne observation requise.

Dans une troisième position, le chirurgien peut voir toujours sans que ses yeux ne quittent les oculaires du microscope tant sur la voie optique du microscope que sur celle de l'endoscope, les deux voies étant confondues en une seule image superposée.

La présente invention a donc pour objet un ensemble comprenant d'une part un microscope constitué d'un binoculaire, d'un corps optique et d'un objectif et d'autre part un endoscope pourvu d'une rallonge et d'un oculaire de sortie qui est caractérisé en ce qu'il comporte, en outre, un module commutateur constitué d'au moins un séparateur associé à un dispositif de réflexion et d'un écran opaque, le dispositif de réflexion et le séparateur étant orientés de telle sorte que leurs normales respectives sont confondues avec la bissectrice que fait le faisceau incident et la direction de l'axe optique considéré, lequel module commutateur est interposé entre le binoculaire et le corps optique du microscope et l'oculaire de sortie de l'endoscope pour permettre à un observateur dont chaque oeil ne quitte pas chaque oculaire du microscope, d'observer à volonté soit la voie optique du microscope, soit la voie optique ou électronique de l'endoscope ou simultanément les deux voies optique du microscope et optique ou électronique ce l'endoscope afin de balayer l'objet à explorer.

La présente invention concerne également les caractéristiques ci-après considérées isolément ou selon toutes leurs combinaisons techniquement possibles :
- le module commutateur est escamotable;
- la distance entre le séparateur et le dispositif de réflexion est égale à 1/√2̅a̅ où a est l'entre-axe c'est-à-dire la distance entre les axes optiques des deux voies du microscope ;
- le séparateur est une lame de verre dont une face a subi un traitement semi-réfléchissant et le dispositif de réflexion est un miroir ;
- les moyens optiques de l'endoscope sont adaptés à ceux du microscope en utilisant un système optique dioptrique ou catadioptrique accordant en dimension et en position les images fournies par l'endoscope ou par le microscope ;
- l'endoscope comporte un oculaire de sortie fournissant une image à l'infini et le microscope procure dans l'espace entre le corps optique et le binoculaire une image à l'infini ;
- le module commutateur comprend un prisme à réflexion interne, un cube séparateur constitué de deux prismes et un écran opaque et est escamoté au moyen d'un moteur d'entraînement ;
- le système optique dioptrique ou catadioptrique est de l'air ;
- le système optique dioptrique ou catadioptrique est un miroir ;
- le système optique dioptrique ou catadioptrique est une combinaison de doublets convergents et divergents et d'un prisme à angle droit ;
- l'écran opaque est une tôle peinte opaque à la lumière.

Divers avantages et caractéristiques de la présente invention ressortiront de la description détaillée ci-après faite en regard des dessins annexés sur lesquels :
Figures 1A et 1B représentent un schéma général de ensemble de l'invention.
Figure 1C illustre la relation de positionnement des éléments optiques du module commutateur.
Figure 2 représente un mode de réalisation avec plusieurs éléments optiques.
Figure 3 est une variante de réalisation de l'appareil selon la Figure 1A.
Figure 4 est une autre variante de l'ensemble de l'invention.
Figure 5 est une coupe verticale d'un mode de réalisation pour escamoter le module commutateur.
Figure 6 est une vue en partie en coupe horizontale et en partie en plan du dispositif de la Figure 5.

Aux dessins annexés où les mêmes symboles de référence se rapportent à des parties analogues, le microscope est désigné par 1 et l'endoscope par 2.

Comme cela est classique, le microscope comprend un binoculaire 3, un corps optique 8 et un objectif 9. L'objet à explorer est symbolisé par la référence 15. Le microscope 1 présente deux oculaires 4 et 5 et des prismes internes 6 et 7. L'endoscope 2 comprend une rallonge 10 qui permet de reporter l'image de sortie de l'endoscope 2 au niveau de l'entrée de l'optique d'adaptation, c'est à dire de l'oculaire de sortie 16 de la rallonge 10 de l'endoscope 2. Une bague de support 11 maintient dans la position désirée l'oculaire de sortie 16 de la rallonge 10 de l'endoscope 2.

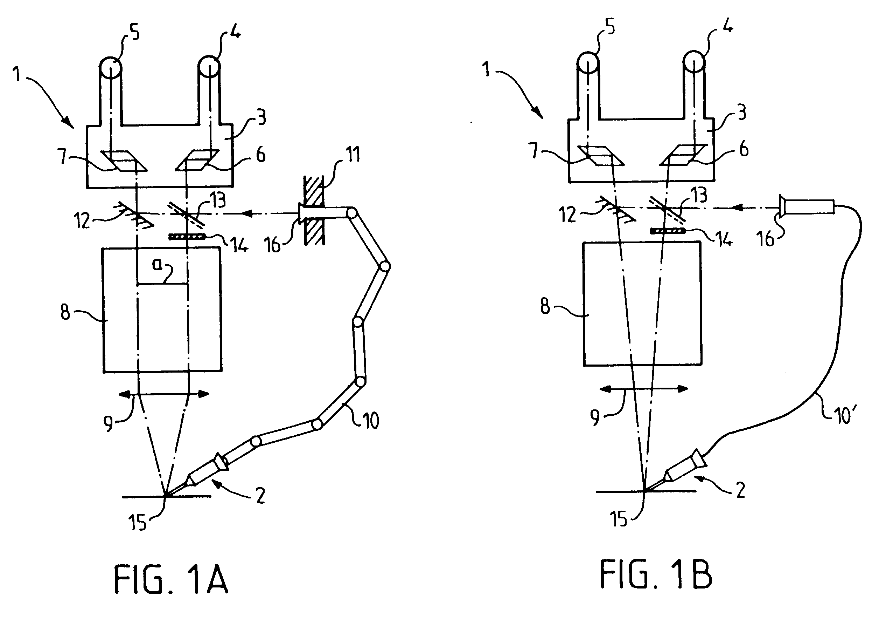

Sur les Figures 1A, 1B, le module commutateur se compose d'un miroir 12, d'un séparateur 13 et d'un écran opaque 14. Le séparateur 13 est une lame de verre dont une face a subi un traitement semi-réfléchissant. Ceci a été matérialisé sur les Figures par des traits pointillés.

Le module commutateur se compose donc dans ce mode de réalisation d'au moins un séparateur 13 associé à un miroir 12. La distance entre le séparateur 13 et le miroir 12 est fonction de l'entre-axe a qui est la distance entre les axes optiques des deux voies. La distance entre le séparateur 13 et le miroir 12 est égale, de préférence, à 1/√2̅a̅. Habituellement, le miroir 12 est un miroir plan et le séparateur est un dioptre plan.

Outre le paramètre de distance entre ces deux éléments, l'autre paramètre à considérer est l'orientation.

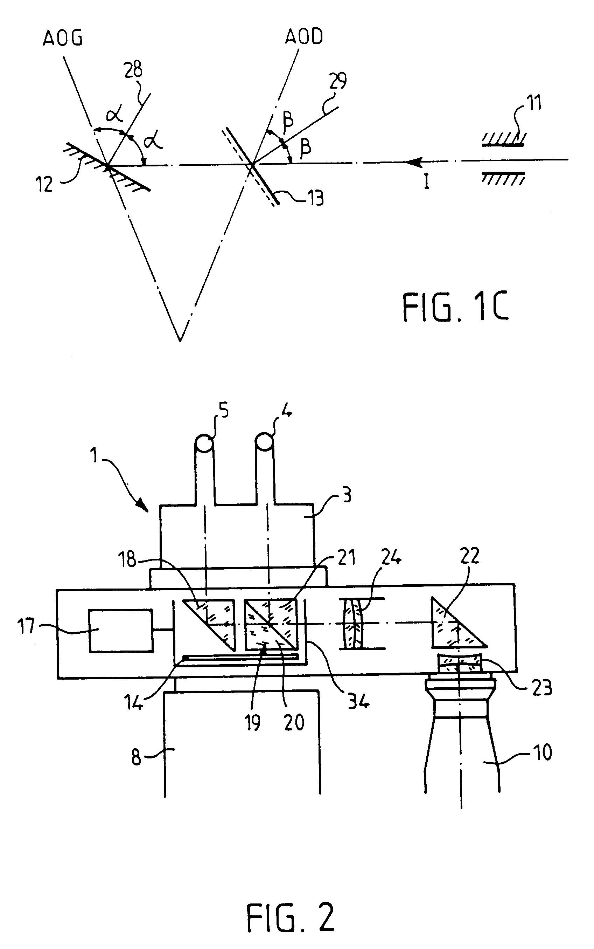

Comme illustré sur la Figure 1C, le miroir 12 et le séparateur 13 sont orientés de sorte que leurs normales respectives 28 et 29 sont confondues avec la bissectrice de l'angle α et β que fait le faisceau incident i provenant de l'endoscope 2 et la direction de l'axe optique considéré. Les moyens optiques de l'endoscope 2 et de sa rallonge 10 sont adaptés à ceux du microscope 1. A cet effet, on utilise un système optique dioptrique ou catadioptrique qui accorde en dimension et en position les images fournies par l'endoscope 2 ou par le microscope 1.

Dans l'exemple illustré sur les Figures 1A et 1B, ce système optique est constitué simplement par de l'air. Dans une telle configuration, l'air constitue un système optique à focale de grossissement 1. L'endoscope 2 comporte aussi un oculaire de sortie 16 qui fournit une image à l'infini tandis que le microscope 1 procure aussi dans l'espace entre le corps optique 8 et le binoculaire 3 une image à l'infini. La référence 10′ symbolise une fibre optique dans le cas où la rallonge 10 renferme une telle fibre optique 10′ et n'est pas constituée uniquement par des ensembles de lentilles et de prismes.

La bague de support 11 est réglable pour avoir les conditions nécessaires et les relations définies.

Sur les Figures 1A et 1B, l'écran opaque 14 est situé sous le séparateur 13 pour occulter la voie droite, c'est à dire celle du séparateur.

Dans cette position, le chirurgien observe l'endoscope, par contraste lorsque l'ensemble du module commutateur est escamoté, le chirurgien voit uniquement avec la voie optique du microscope. L'occultation est obtenue, de préférence, par tout moyen approprié qui est actionné par une pédale, ce qui laisse les mains libres à l'utilisateur.

La Figure 2 montre un exemple pratique de mise en oeuvre de la présente invention.

Le module commutateur se compose d'un prisme à réflexion interne 18, d'un cube séparateur 19 constitué de deux prismes 20 et 21 et de l'écran opaque 14.

Les primes 18, 20 et 21 sont des prismes à angles droits. Les deux prismes 20 et 21 sont, par exemple, collés et ont subi un traitement semi-réfléchissant. Ce module commutateur est entraîné par un moteur électrique 17 lorsque l'on veut escamoter tout ou partie des éléments 18, 19, 14. Lorsqu'on escamote l'ensemble, l'utilisateur dont les yeux sont appliqués contre les oculaires 4 et 5 voit par l'intermédiaire du miscroscope. On peut aussi escamoter, si désiré, uniquement l'écran opaque 14.

La position modifiée de la rallonge 10 de l'endoscope vis à vis du microscope 1 implique l'utilisation d'un doublet divergent 23, d'un doublet convergent 24 et l'interposition entre ces doublets 23, 24 d'un prisme à angle droit 22.

A titre d'exemple, le doublet divergent 24 a une focale de - 50, le doublet convergent 23 a une focale de 100, l'ensemble est afocal de grossissement 0,5.

Avec les modes de réalisations représentés sur les dessins, trois positions sont obtenues :
Dans la première position, le module commutateur n'est pas escamoté, auquel cas l'utilisateur dont les yeux sont appliqués sur les oculaires 4 et 5 du microscope voit selon la voie optique de l'endoscope.

Dans la deuxième position, l'ensemble du module commutateur est escamoté, auquel cas le chirurgien observe par l'intermédiaire des oculaires 4 et 5 la voie optique du microscope.

Dans la troisième position, en escamotant simplement l'écran opaque 14, l'utilisateur peut voir sur les deux voies microscope et endoscope confondues en une seule image, il s'agit là d'un phénomène de mixage.

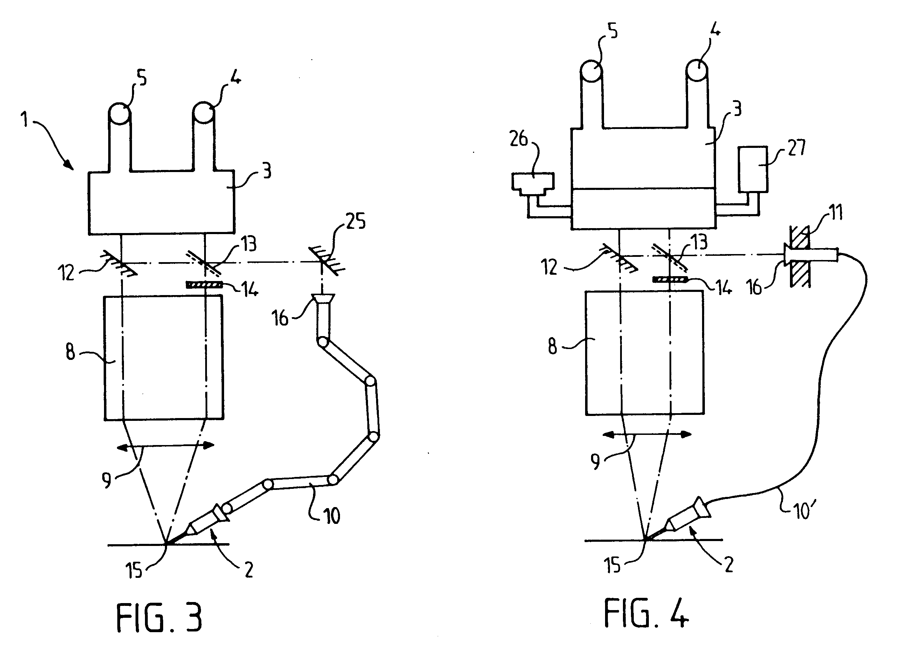

Sur la Figure 3, on a illustré une variante de mise en oeuvre dans laquelle on utilise un miroir 25 avant l'oculaire de sortie 16 de la rallonge 10 de l'endoscope 2.

Le miroir 25 est nécessaire du fait de la position de l'oculaire de sortie 16.

La Figure 4 illustre la prévision d'accessoires photographiques 26 et d'accessoires vidéo 27 qui peuvent être montés en amont du corps optique 8 du microscope 1.

Sur la Figure 1C, l'axe optique gauche a été désigné par AOG tandis que l'axe optique droit a été désigné par AOD.

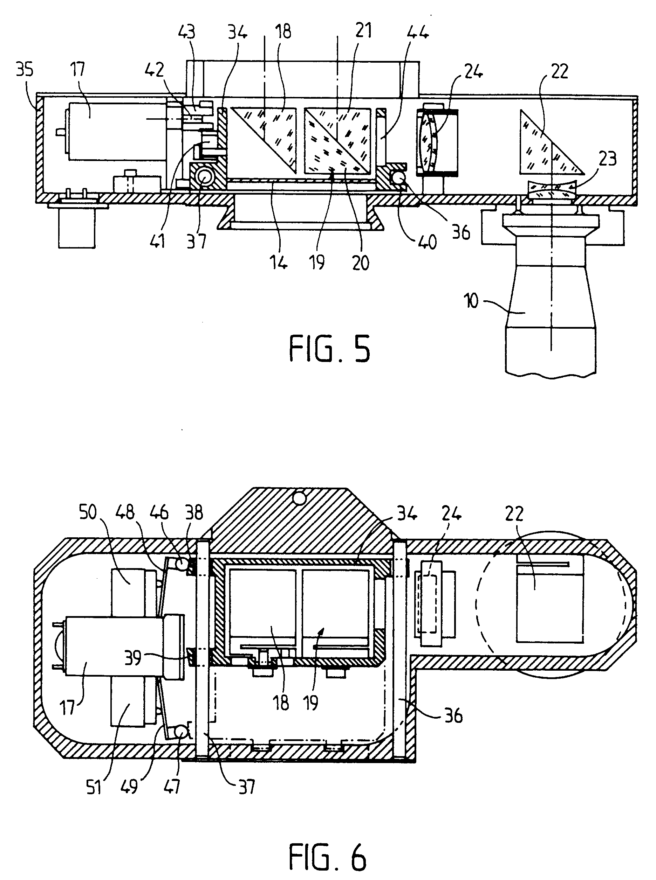

En référence aux Figures 5 et 6, on explique ci-après un exemple de déplacement du commutateur de la présente invention constitué par le prisme à réflexion interne 18 et le cube séparateur 19 à deux prismes 20 et 21.

Ces deux éléments sont montés dans un chariot 34 qui est monté coulissant transversalement par rapport au châssis 35 sur deux colonnes latérales et parallèles 36, 37 lesquelles sont fixées au châssis 35. Le guidage sur la colonne 37 est assuré par des manchons co-axiaux 38, 39 solidaires de la base du chariot 34 et le guidage sur la colonne 36 est assuré par une chape 40 solidaire de la base du chariot 34.

Une crémaillère 41 est fixée sur un côté du chariot 34 au-dessus des manchons 38, 39 et de la colonne 37 et parallèlement à l'axe de cette dernière. Le moteur électrique réversible 17 fixé dans le châssis 35 d'un côté du chariot 34 entraîne en rotation par son arbre de sortie 42 perpendiculaire à la crémaillère 31 et aux colonnes 37, 36, un pignon 43 dont les dents sont en prise avec celles de la crémaillère 41. Ainsi, lorsque le pignon 43 est entraîné en rotation dans l'un ou l'autre sens par le moteur 17, le chariot 34 et les éléments optiques qu'il porte sont translatés dans l'un ou l'autre sens sur les colonnes 36 et 37 et entre deux positions l'une sur la voie optique de l'endoscope, l'autre escamotée de la voie optique de l'endoscope.

Le chariot 34 présente une fenêtre 44 du côté opposé au moteur 17.

L'écran opaque 14 est monté coulissant dans des glissières à l'intérieur de la base du chariot 34 et peut être escamoté à volonté lorsqu'on désire une vision sur les deux voies optiques.

Pour couper l'alimentation du moteur dans l'un ou l'autre sens de rotation, lorsque le chariot 34 arrive à l'une de ses positions extrêmes sur le châssis 35, l'un des manchons 38, 39 actionne un contacteur de fin de course comprenant un toucheau 46, 47 monté à l'extrêmité d'un bras élastique 48, 49 monté en saillie latérale sur le moteur 17 et dont la déformation ou le déplacement vient actionner un micro-interrupteur 50, 51 accouplé à ce dernier pour sa commande.

## Revendications

1. Ensemble comprenant d'une part un microscope (1) constitué d'un binoculaire (3), d'un corps optique (8) et d'un objectif (9) et d'autre part un endoscope (2) pourvu d'une rallonge (10) et d'un oculaire de sortie (16), caractérisé en ce qu'il comporte, en outre, un module commutateur (12, 13, 14, 18, 19, 14) constitué d'au moins un séparateur (13, 19) associé à un dispositif de réflexion (12, 18) et d'un écran opaque (14), le dispositif de réflexion (12, 18) et le séparateur (13, 19) étant associés de telle sorte que leurs normales respectives (28, 29) sont confondues avec la bisectrice que fait le faisceau incident (I) et la direction de l'axe optique considéré, lequel module commutateur est interposé entre le binoculaire (3) et le corps optique (8) du microscope (1) et l'oculaire de sortie (16) de l'endoscope (2) pour permettre à un observateur dont chaque oeil ne quitte pas chaque oculaire (4, 5) du microscope (1) d'observer à volonté soit la voie optique du microscope (1), soit la voie optique ou électronique de l'endoscope (2) ou simultanément les deux voies optique du microscope (1) et optique ou électronique de l'endoscope (2) afin de balayer l'objet à explorer(15).

2. Ensemble selon la revendication 1, caractérisé en ce que le module commutateur (12, 13, 14, 18, 19, 14) est escamotable.

3. Ensemble selon la revendication 2, caractérisé en ce que la distance entre le séparateur (13, 19) et le dispositif de réflexion (12, 18) est égale à 1/√2̅a̅ où a est l'entre-axe c'est à dire la distance entre les axes optiques des deux voies du microscope (1).

4. Ensemble selon l'une quelconque des revendications 1 à 3, caractérisé en ce que le séparateur (13) est une lame de verre dont une face a subi un traitement semiréfléchissant et le dispositif de réflexion (12) est un miroir.

5. Ensemble selon l'une quelconque des revendications 1 à 4, caractérisé en ce que les moyens optiques de l'endoscope (2) sont adaptés à ceux du microscope (1) en utilisant un système optique dioptrique ou catadioptrique accordant en dimension et en position les images formées par l'endoscope (2) ou par le microscope (1).

6. Ensemble selon l'une quelconque des revendications 1 a 4, caractérisé en ce que l'endoscope (2) comporte un oculaire de sortie (16) fournissant une image a l'infini et le microscope (1) procure dans l'espace entre le corps optique (8) et le binoculaire (3) une image à l'infini.

7. Ensemble selon la revendication 1, caractérisé en ce que le module commutateur comprend un prisme à réflexion interne (18), un cube séparateur (19) constitué de deux prismes (20, 21) et un écran opaque (14) et est escamoté au moyen d'un moteur d'entraînement (17).

8. Ensemble selon l'une quelconque des revendications 1 a 7, caractérisé en ce que le système optique dioptrique ou catadioptrique est de l'air.

9. Ensemble selon l'une quelconque des revendications 1 à 7, caractérisé en ce que le système optique dioptrique ou catadioptrique est un miroir (25).

10. Ensemble selon l'une quelconque des revendications 1 a 7, caractérisé en ce que le système optique dioptrique ou catadioptrique est une combinaison de doublets convergents (24), divergents (23) et d'un prisme à angle droit.

11. Ensemble selon l'une quelconque des revendications 1 a 10, caractérisé en ce que l'écran opaque (14) est une tôle peinte opaque à la lumière.

## Claims

1. Assembly comprising, on one hand, a microscope (1) consisting of a binocular (3), an optical body (8) and an objective (9), and, one the other hand, an endoscope (2) provided with an extension (10) and an outlet ocular (16), characterized in that it further comprises a commutating modulus (12, 13, 14, 18, 19, 14), consisting of at least one separator (13, 19) associated to a reflecting device (12,18), and of an opaque screen (14), the reflecting device (12,18) and the separator (13, 19) being associated so that their respective normals (28, 29) are united together with the bisectrix between the incident beam (I) and the direction of the relative optical axis, said commutating modulus is placed between the binocular (3) and the optical body (8) of microscope (1) and the outlet ocular (16) of endoscope (2) as to enable an observer having stationary eyes on each ocular (4, 5) of the microscope (1), respectively, to willingly observe either on the optical way of the microscope (1) or on the optical or electronic way of the endoscope (2) or simultaneously both on the optical way of the microsope (1) and on the optical or electronic way of the endoscope (2) on purpose to scan the object (15) to be investigated.

2. Assembly according to claim 1, characterized in that the commutating modulus (12, 13, 14, 18, 19, 14) is retractable.

3. Assembly according to claim 2, characterized in that the distance between the separator (13, 19) and the reflecting device (12, 18) is equal to 1/√2̅a̅ where a is the axis gap i.e. the distance between the optical axes of both ways of the microscope.

4. Assembly according to any one of claims 1 to 3, characterized in that the separator (13) is a glass slab one-face of which has been subjected to a semi-reflecting treatment and the reflecting device (12) is a mirror.

5. Assembly according to any one of claims 1 to 4, characterized in that the optical means of the endoscope (2) are adapted to that of the microscope (1) while using a dioptric or catadioptric optical system which dimensionally and positionally brings into accord both images formed by the endoscope (2) or the microscope (1).

6. Assembly according to any one of claims 1 to 4, characterized in that the endoscope (2) comprises an outlet ocular (16) providing an image for infinity and the microscope (1) provides an image for infinity in the space gap between the optical body (8) and the binocular (3).

7. Assembly according to claim 1, characterized in that the commutating device comprises an internal reflecting prism (18), a separating cube (19) consisting of two prisms (20, 21) and an opaque screen (14) and is retracted by means of a driving motor (17).

8. Assembly according to any one of claims 1 to 7, characterized in that the dioptric or catadioptric optical system consists of air medium.

9. Assembly according to any one of claims 1 to 7, characterized in that the dioptric or catadioptric optical system is a mirror (25).

10. Assembly according to any one of claims 1 to 7, characterized in that the dioptric or catadioptric optical system is a combination of convergent (24) and divergent (23) lenses and of a reflecting prism.

11. Assembly according to any one of claims 1 to 10, characterized in that the opaque screen (14) is a metal sheet which has been painted as to be light-opaque.

## Patentansprüche

1. Anordnung, die einerseits ein Mikroskop (1), bestehend aus einem Binokular (3), einem optischen Körper (8) und einem Objektiv (9), und andererseits ein Endoskop (2) aufweist, das mit einer Verlängerung (10) und einem Ausgangsokular (16) versehen ist, dadurch gekennzeichnet, daß sie ferner ein Schaltmodul (12, 13 14; 18, 19, 14) aufweist, das aus wenigstens einem Strahlteiler (13, 19) besteht, der einer Reflektionsvorrichtung (12, 18) und einem opaken Schirm (14) zugeordnet ist, wobei die Reflektionsvorrichtung (12, 18) und der Strahlteiler(13, 19) einander derart zugeordnet sind, daß ihre jeweiligen Normalen (28, 29) mit der die von dem einfallenden Strahl (I) und der Richtung der jeweiligen optischen Achse gebildeten Winkel Halbierenden zusammenfallen, wobei das Schaltmodul zwischen dem Binokular (3) und dem optischen Körper (8) des Mikroskops (1) und dem Ausgangsokular (16) des Endoskops (2) angeordnet ist, damit ein Beobachter, ohne daß seine Augen die Okulare (4, 5) des Mikroskops (1) verlassen, zum Abtasten des zu untersuchenden Objekts (15) wahlweise entweder den optischen Weg des Mikroskops (1) oder den optischen oder elektronischen Weg des Endoskops (2) oder gleichzeitig die beiden optischen Wege des Mikroskops (1) und den optischen oder elektronischen weg des Endoskops (2) beobachten kann.

2. Anordnung nach Anspruch 1, dadurch gekennzeichnet, daß das Schaltmodul (12, 13, 14; 18, 19, 14) zusammenklappbar ist.

3. Anordnung nach Anspruch 2, dadurch gekennzeichnet, daß der Abstand zwischen dem Strahlteiler (13, 19) und der Reflektionsvorrichtung (12, 18) gleich 1/√2̅a̅ ist, wobei a den Achsabstand bezeichnet, das heißt, den Abstand zwischen den optischen Achsen der beiden Wege des Mikroskops (1).

4. Anordnung nach einem der Ansprüche 1 bis 3, dadurch gekennzeichnet, daß der Strahlteiler (13) eine Glasplatte ist, deren eine Seite halbreflektierend bearbeitet iat, und die Reflektionsvorrichtung (12) ein Spiegel ist.

5. Anordnung nach einem der Ansprüche 1 bis 4, dadurch gekennzeichnet, daß die optischen Einrichtungen des Endoskops (2) unter Verwendung eines dioptrischen oder katadioptrischen optischen Systems, das die von dem Endoskop (2) oder dem Mikroskop (1) erzeugten Bilder größen- und positionsmäßig abstimmt, an diejenigen des Mikroskops (1) angepaßt sind.

6. Anordnung nach einem der Ansprüche 1 bis 4, dadurch gekennzeichnet, daß das Endoskop (2) ein Ausgangsokular (16) aufweist, das ein Bild im Unendlichen liefert, und das Mikroskop (1) in dem Raum zwischen dem optischen Körper (8) und dem Binokular (3) ein Bild im Unendlichen erzeugt.

7. Anordnung nach Anspruch 1, dadurch gekennzeichnet, daß das Schaltmodul ein Prisma (18) mit innerer Reflektion, einen aus zwei Prismen (20, 21) bestehenden Strahlteilerkubus (19) und einen opaken Schirm (14) enthält und mittels eines Antriebsmotors (17) eingeklappt wird.

8. Anordnung nach einem der Ansprüche 1 bis 7, dadurch gekennzeichnet, daß das dioptrische oder katadioptrische optische System Luft ist.

9. Anordnung nach einem der Ansprüche 1 bis 7, dadurch gekennzeichnet, daß das dioptrische oder katadioptrische optische System ein Spiegel ist.

10. Anordnung nach einem der Ansprüche 1 bis 7, dadurch gekennzeichnet, daß das dioptrische oder katadioptrische optische System eine Kombination aus konvergierenden (24), und divergierenden Doppellinsen (23) und einem rechtwinkligen Prisma ist.

11. Anordnung nach einem der Ansprüche 1 bis 10, dadurch gekennzeichnet, daß der opake Schirm (14) ein lichtundurchlässig gefärbtes Blech ist.
